# EUROPEAN PATENT APPLICATION

(11) **EP 4 706 608 A1**
(43) Date of publication of application: **11.03.2026**
(21) Application number: 25200371.0
(22) Date of filing: 04.09.2025
(51) Int. Cl.: A61F 9/008

(54) **OPHTHALMOLOGICAL LASER TREATMENT DEVICE**

(30) Priority: 06.09.2024 CH 9642024
(71) Applicant: Ziemer Ophthalmic Systems AG, 2562 Port (CH)
(72) Inventor: RATHJEN, Christian, 28197 Bremen (DE); STEINLECHNER, Michael, 8006 Zürich (CH)
(74) Representative: Rentsch Partner AG

(57) **Abstract**

The present disclosure relates to an ophthalmological laser treatment device (1), comprising: an optical system (2), a scanner system (3) coupled to the optical system (2), an imaging system (4), and an electronic circuit (5) configured to: receive one or more images, generate an eye tissue modification score, using the one or more images, the eye tissue modification score being indicative of an extent of modification of the eye tissue, and generate one or more feedback signals depending on the eye tissue modification score.

## Description

### FIELD OF THE DISCLOSURE

The present disclosure relates to an ophthalmological laser treatment device.

### BACKGROUND OF THE DISCLOSURE

Ophthalmological laser treatment systems are known which incorporate cameras such that operators of the treatment system, such as eye surgeons, are able to properly set up the treatment system prior to eye treatment and also to view a camera feed during surgery, for example on a monitor.

Known systems may use such cameras to monitor a focal position of the treatment laser beam, or a focal spot size, during laser treatment.

### SUMMARY OF THE DISCLOSURE

It is an object of the disclosure and embodiments disclosed herein to provide an ophthalmological laser treatment device.

In particular, it is an object of the disclosure and embodiments disclosed herein to provide an ophthalmological laser treatment device, as well as a method for operating an ophthalmological laser treatment device, which improves on the prior art by offering new monitoring capabilities.

The present disclosure relates to an ophthalmological laser treatment device. The ophthalmological laser treatment device comprising an optical system. The optical system comprises a plurality of optical elements configured to focus a treatment laser beam onto a focal spot in eye tissue of an eye. The ophthalmological laser treatment device comprises a scanner system. The scanner system is coupled to the optical system and configured to move at least one of the optical elements of the optical system such that the focal spot in the eye tissue moves according to a scan pattern. The scan pattern may be pre-defined. The ophthalmological laser treatment device comprises an imaging system. The imaging system is optically coupled to the optical system and configured to capture one or more images of the area surrounding the focal spot. The ophthalmological laser treatment device comprises an electronic circuit. The electronic circuit is configured to receive, from the imaging system, the one or more images. The electronic circuit is configured to generate an eye tissue modification score, using the one or more images. The eye tissue modification score is indicative of an extent of modification of the eye tissue achieved by moving the focal spot of the treatment laser beam in the eye tissue. The electronic circuit is configured to generate one or more feedback signals depending on the eye tissue modification score.

The eye tissue may be modified due to it being disrupted and/or ablated by the treatment laser, for example.

The optical system defines an image area. The image area lies in an image plane of the optical system. The image area corresponds to the field of view of the optical system. The image area may be circular (referred to as an image circle herein) due to a circular aperture of the optical system. The image area may have other shapes however, depending on the particulars of the optical system and/or additional shutters or diaphragms in the optical path. The imaging system therefore is configured to capture one or more images of the area surrounding the focal spot. The focal spot is located within the image area and the images of the image area therefore include at least part of the image area.

In an embodiment, the scanner system may optionally comprise a slow scanner or main scanner configured to move the image area and thereby also the focal spot according to the scan pattern and may optionally comprise a fast scanner or auxiliary scanner which superimposes, onto the scan pattern, an additional movement of the focal spot at a speed which is faster than the speed at which the slow scanner moves the focal spot.

In an embodiment, the electronic circuit is configured to identify eye tissue changes in the one or more images. The eye tissue changes are due to photo modification of the eye tissue by the treatment laser beam. The eye tissue changes relate to one of more of: gas created inside the eye tissue, a change in a refractive index of the eye tissue, a change in the polarization of light due to the modification of the eye tissue, and/or a change in the color of the eye tissue. The electronic circuit is configured to generate the eye tissue modification score using the identified eye tissue changes.

In an embodiment, the imaging system is configured to capture the one or more images during activation of the treatment laser. In other words, the imaging system is designed to capture the images at the same time as the treatment laser beam is turned on and directed into the eye. Thereby, the images are captured simultaneously with the treatment laser beam treating the eye and in real time during treatment process. The electronic circuit is configured to generate the eye tissue modification score using the one or more images captured during activation of the treatment laser. Thereby, the eye tissue modification score is indicative of the dynamic process of eye tissue modification. This is advantageous because the images can be captured without interrupting the treatment, in particular without shutting off the treatment laser beam or stopping it from being directed into the eye and therefore, does not impede treatment or slow it down. The tissue modification score being indicative of the dynamic, i.e. real time, process of eye tissue modification, the tissue modification score reflects or is indicative of not only the final result of eye tissue treatment, for example relating to a cut depth or ablation result, but indicative of instantaneous and transient processes which occur during eye tissue modification.

In an embodiment, the imaging system is configured to capture one or more two-dimensional images of the eye tissue formed in an image plane of the optical system. The image plane of the optical system has a surface normal vector parallel to the optical axis of the optical system. In other words, the imaging system records the top-down view of the area of the eye being treated, as opposed to depth slice or scan.

In an embodiment, the electronic circuit is configured to determine global image properties in the one or more images and to generate the eye tissue modification score depending on the global image properties. The global image properties are properties which are determined using the whole, or at least substantially all, of each of the one or more images, in particular the part of the image representative of the eye tissue.

In an embodiment, the image processing comprises determining local image properties in the one or more images.

In an embodiment, the imaging system is optically coupled to the optical system by way of a beam splitter such as to capture the one or more images of the eye tissue substantially coaxially through the optical system.

In an embodiment, the imaging system captures a plurality of images in which at least some adjacent images partially overlap each other. The eye tissue modification score is generated for a particular image (or a particular area of eye tissue captured by the particular image) using, in addition to the particular image itself, one or more further images adjacent to the particular image. In particular, the images partially overlap each other in image overlap areas and the eye tissue modification score is generated for a particular image or a particular area of eye tissue captured by the particular image using image data from the one or more image overlap areas of the further images adjacent to the particular image.

In an embodiment, at least some of the adjacent images comprise overlapping areas of eye tissue, wherein the adjacent images overlap each other by at least 90%, preferably at least 95%.

In an embodiment, the electronic circuit is further configured to associate the one or more received images with one or more positions in the eye, respectively.

In an embodiment, the evaluation module is further configured to generate an eye tissue modification map. The eye tissue modification map is generated using the eye tissue modification score(s). The eye tissue modification map comprises, for each of a plurality of positions in the eye, an indicator of whether the eye tissue has been modified.

The eye tissue modification map may be included as part of the feedback signal(s).

In an embodiment, the electronic circuit is further configured to provide, to a display, an image of the eye including an overlaid rendering of the eye tissue modification map.

In an embodiment, the feedback signal is indicative of eye tissue which, after having been targeted by the focal spot, was not modified.

In an embodiment, the electronic circuit is further configured to adjust treatment parameters of the ophthalmological laser treatment device depending on the eye tissue modification score. The treatment parameters include one or more of: the scan pattern, a beam power, a repetition rate of the treatment laser beam, and/or a focal spot size.

In an embodiment, the electronic circuit is further configured to generate a mosaic image using the one or more images, and to generate, for a plurality of positions in the mosaic image, the eye tissue modification score.

In an embodiment, the electronic circuit described herein is configured to generate a mosaic image using a plurality of received images. The electronic circuit may optionally be further configured to generate the eye tissue modification score. In particular, the electronic circuit is configured to receive, from the imaging system, a plurality of images. The electronic circuit is configured to process each of the plurality of images to generate a mosaic image piece. The electronic circuit is configured to generate, using the plurality of mosaic image pieces, a mosaic image.

The electronic circuit may be configured to generate the feedback signal to comprise the mosaic image.

The electronic circuit may be configured to determine, for each of the plurality of images, a position. The position of each image may relate to its position relative to, or with respect to, the scan pattern, one or more other images, and/or the eye. The position may be determined using a time-stamp of the image, the scan pattern, the treatment model, a position of the optical system and/or a position of the scanner system. The electronic circuit is configured to generate the mosaic image further using the position of each image.

The electronic circuit may be configured to determine, for each of the plurality of images, a rotational angle of the image and may rotate the image about the rotational angle. The rotational angle may be determined using a time-stamp of the image and/or the scan pattern. The electronic circuit may be configured to generate the mosaic image further using the rotated image.

In an embodiment, the plurality of images comprises a sequence of overlapping images. The electronic circuit is configured to generate, using the sequence of overlapping images, a corresponding sequence of non-overlapping, for example touching, mosaic image pieces. The electronic circuit is configured to generate the mosaic image using the sequence of non-overlapping mosaic image pieces.

In an embodiment, the plurality of images may comprise a sequence of non-overlapping images. The mosaic image pieces generated therefrom may therefore also be non-overlapping. The mosaic may be generated such that gaps between the mosaic image pieces are maintained. The gaps may be rendered as transparent areas, for example such that when displayed a background is visible through the gaps. The background may be an image of the eye. The gaps may alternatively be filled by interpolation between the adjacent mosaic image pieces.

In addition to an ophthalmological laser treatment device, the present disclosure also relates to a method for operating an ophthalmological laser treatment device, wherein the ophthalmological laser treatment device comprises an optical system including a plurality of optical elements, a scanner system coupled to the optical system, an imaging system and an electronic circuit. The electronic circuit is configured to perform a number of steps of the method. The steps include focusing, using the optical system, a treatment laser beam onto a focal spot in eye tissue of an eye. The steps include moving, using the scanner system, at least one of the optical elements such that the focal spot in the eye tissue moves according to a scan pattern. The steps include capturing, using the imaging system, one or more images of an area surrounding the focal spot. The steps include receiving, from the imaging system, the one or more images. The steps include generating an eye tissue modification score, using the one or more images. The eye tissue modification score is indicative of an extent of modification of the eye tissue achieved by moving the focal spot of the treatment laser beam in the eye tissue. The steps include generating, one or more feedback signals depending on the eye tissue modification score.

In an embodiment, the electronic circuit performs a method for generating a mosaic image using a plurality of received images. The method may optionally further comprise generating the eye tissue modification score. In particular, the method comprises receiving, from the imaging system, a plurality of images. The method comprises processing each of the plurality of images to generate a mosaic image piece. The method comprises generating, using the plurality of mosaic image pieces, a mosaic image.

In addition to an ophthalmological laser treatment devices and the methods for operating an ophthalmological laser treatment device, the present disclosure also relates to a computer program product. The computer program product comprises computer program code which, when the computer program code is executed by an electronic circuit of an ophthalmological laser treatment device, causes the electronic circuit to perform the steps of one of the methods as described herein. The computer program product may be stored on a non-transitory memory.

### BRIEF DESCRIPTION OF THE DRAWINGS

The herein described disclosure will be more fully understood from the detailed description given herein below and the accompanying drawings, which should not be considered limiting to the invention described in the appended claims. The drawings in which:
- Fig. 1: shows a block diagram illustrating schematically an ophthalmological laser treatment device and the components thereof;
- Fig. 2: shows a schematic diagram of an embodiment of an ophthalmological laser treatment device according to the disclosure;
- Fig. 3: shows a schematic diagram of an embodiment of an ophthalmological laser treatment device according to the disclosure in which the treatment laser beam travels along an arm between a base station and an application head;
- Fig. 4: shows a schematic diagram of the focal spot formed by the treatment laser beam moving in a scan direction according to a scan pattern, with light scattered in the eye tissue being recorded by the imaging system;
- Fig. 5: shows a schematic diagram of the view through the optical system, in particular showing an area of eye tissue along with a treatment laser beam focal spot and modified eye tissue in the wake of the focal spot;
- Fig. 6: shows a schematic diagram of the view through the optical system, similar to Fig. 4, however further showing multiple focal spots of the treatment laser beam situated along the fast scan line;
- Fig. 7: shows a visual rendering of a view through the optical system of eye tissue in three different stages of treatment, the first including eye tissue after treatment in which ambient light is scattered by the modified eye tissue, the second including eye tissue during treatment in which half of the viewed area has been modified and half has yet to be treated, and the third including eye tissue prior to treatment;
- Fig. 8: shows a schematic diagram of a view through the optical system including a black and white image of eye tissue during treatment in which, on the left half of the image the modified tissue is evident and on the right half the unmodified tissue is shown;
- Fig. 9: shows a diagram including an image of eye tissue along with the columnwise brightness distribution of the image, showing how the eye tissue modification score may be generated based on the image;
- Fig. 10: shows a schematic diagram of an eye tissue modification map for a part of the eye tissue which has undergone treatment;
- Fig. 11: shows a schematic diagram of an eye including a rendering of an eye tissue modification map superimposed on the eye;
- Fig. 12: shows a schematic diagram of an eye including a rendering of an eye tissue modification map superimposed on the eye, including the scan pattern;
- Fig. 13: shows a schematic diagram of a plurality of images recorded in sequence, illustrating overlapping areas between adjacent images;
- Fig. 14: shows a flow diagram illustrating a method for generating an eye tissue modification score;
- Fig. 15: shows a flow diagram illustrating a further method for generating an eye tissue modification score;
- Fig. 16: shows a block diagram illustrating possible steps performed to generate the eye tissue modification score;
- Fig. 17: shows a flow diagram illustrating a method for generating an eye tissue modification map; and
- Fig. 18: shows a flow diagram illustrating a method for generating a mosaic image.

### DESCRIPTION OF THE EMBODIMENTS

Reference will now be made in detail to certain embodiments, examples of which are illustrated in the accompanying drawings, in which some, but not all features are shown. Indeed, embodiments disclosed herein may be embodied in many different forms and should not be construed as limited to the embodiments set forth herein; rather, these embodiments are provided so that this disclosure will satisfy applicable legal requirements. Whenever possible, like reference numbers will be used to refer to like components resp. modules or parts.

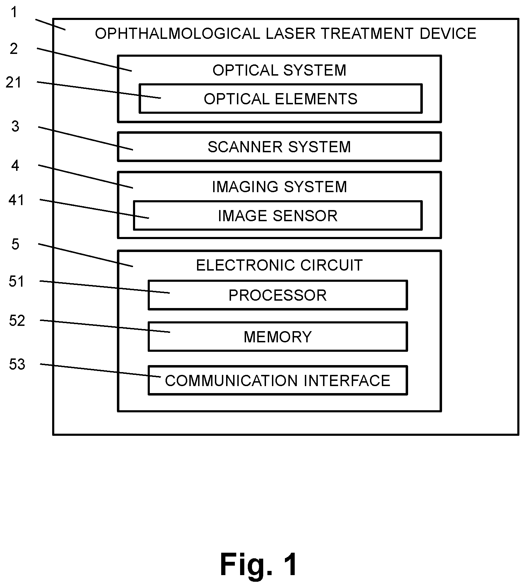

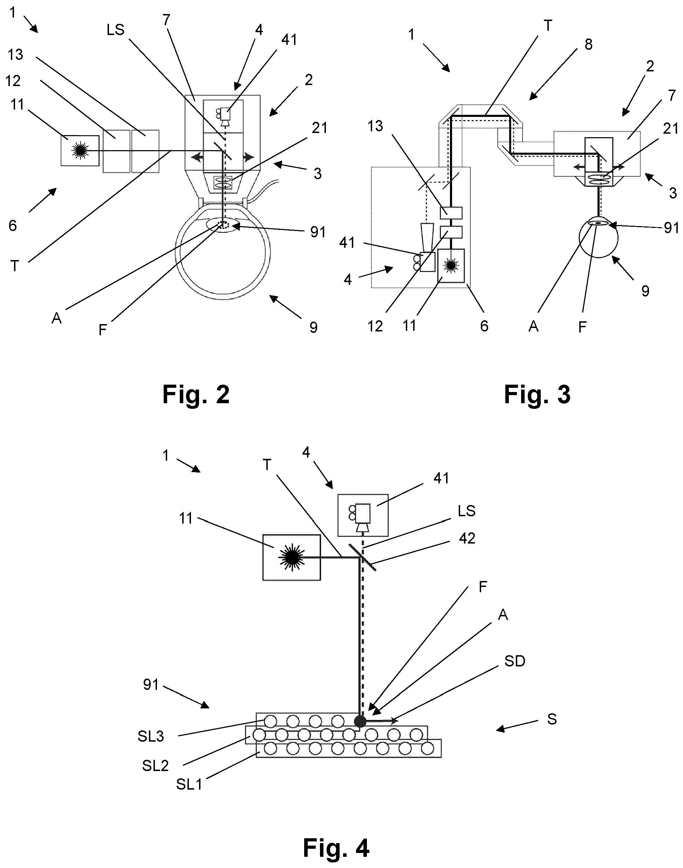

**Figure 1** shows a block diagram diagram illustrating schematically an ophthalmological laser treatment device 1. Fig. 1 and in particular also Figs. 2 and 3 schematically illustrate modules and/or elements of various embodiments of the ophthalmological laser treatment device 1 and give an exemplary sequence or arrangement of modules and/or elements, including modules and/or elements in a beam path. The skilled person understands that at least some modules and/or elements shown in a particular Figure may be combined with modules and/or elements shown in another Figure.

The ophthalmological laser treatment device 1 may be configured as a fixed or mobile apparatus. The ophthalmological laser treatment device 1 has a treatment laser source 11 which generates a treatment laser beam T.

In another example, the treatment laser source 11 is configured to generate an infrared treatment laser beam T having a wavelength of between 780 nm and 1100 nm. For example, the treatment laser source 11 comprises a solid-state laser, such as a Ytterbium based laser. The treatment laser source 11 will not be described in further detail, however the skilled person is aware that the treatment laser source 11 can comprise, for example, a gain medium, a laser resonator, a laser pump, a pulse generating element, cavity mirrors, coupling mirrors, wavelength tuners, and/or a frequency converter (including one or more non-linear optical crystals).

Depending on the embodiment, the treatment laser beam T is a pulsed laser beam.

In an embodiment, the treatment laser source 11 is configured to generate femtosecond laser pulses, which have pulse widths of typically from 10 fs to 1000 fs (1 fs = 10⁻¹⁵ s).

The repetition rate of the treatment laser source 11 is typically at least two orders of magnitude greater than the frame rate of the imaging system 4. For example, the treatment laser source 11 may have a 100 KHz repetition, while the imaging system 4 has a frame rate of 1000 Hz.

The treatment laser source 11 may generate the treatment laser beam T having a defined beam power and/or repetition rate.

Downstream of the treatment laser source 11, the ophthalmological laser treatment device 1 includes an optional laser attenuator 12 configured to attenuate the treatment laser beam T.

Depending on the embodiment, a beam shaper 13 is included in the ophthalmological laser treatment device 1, arranged downstream from the treatment laser source 11. The beam shaper is configured to control the laser beam profile, in particular to redistribute the irradiance of the treatment laser beam T to attain a desired laser beam profile at the focal spot in the eye 9.

The aforementioned components of the ophthalmological laser treatment device 1 may be arranged in a base station, which is a fixed or mobile apparatus or unit.

The ophthalmological laser treatment device 1 further comprises, downstream from the laser source 11, an optical system 2 and a scanner system 3 which are designed to focus and scan the treatment laser beam T, respectively. These components are typically arranged in a part of the ophthalmological laser treatment device 1 close to the eye 9 of a patient during treatment, in particular in close proximity to a patient interface. They may, for example, be connected or integrated into a so-called application head. These components may alternatively be arranged in a gantry. The gantry is a part or section of the ophthalmological laser treatment device 1 which extends over the patient during treatment.

The ophthalmological laser treatment device 1 may comprise a fiber optic cable configured to guide the treatment laser beam at least part of the way between the treatment laser beam source 11 or from a component arranged downstream of the treatment laser beam source 11 (which may be housed in the base station 2), to the optical system 2.

As shown in Fig. 3, in an embodiment, the ophthalmological laser treatment device 1 can comprise an articulated arm 8 configured to guide the treatment laser beam T from the base station 6. The articulated arm 8 may comprise one or more mirrors to provide a beam path for the treatment laser beam T.

The optical system 2 comprises a plurality of optical elements 21. The optical elements 21 include, for example, one or more lenses for focusing the treatment laser beam T onto a focal spot F in the eye 9. The optical system 2 may be adjustable, in particular by moving one of the optical elements 21 relative to other optical elements 21, such as to move the focal spot F in a z-direction (the z-direction is parallel to the beam path of the treatment laser beam T). The optical system 2 may further be adjustable such as to adjust a size of the focal spot F.

The optical system 2 is a partial field of view optical system 2 in that it is designed such that its field of view, which corresponds to the image circle of the optical system 2, only partially views the eye 9 during treatment. In particular, the optical system 2 on partially views the eye tissue 91 to be treated according to the treatment model. The eye tissue 91 to be treated includes, for example, a lens of the eye 9 and/or a cornea of the eye 9.

The optical system 2 does not and is not capable of viewing the entire eye 9 or the entire eye tissue 91 and must be moved across the eye 9 by the scanner system 3 to reach all areas of the eye tissue 91 to be treated. For example, the image circle of the optical system 2 in the eye 9 has a radius on the order of 1 millimeter, for example a radius in a range between 0.1 mm and 3 mm, preferably between 0.5 mm and 2 mm.

The optical system 2, as it is not required to view the entire eye 9 or the entire eye tissue 91 to be treated, has a more compact design than a conventional full field of view optical system. This allows for the optical system to be integrated into a compact housing, which housing may for example form part of an application head of the ophthalmological laser treatment device 1 separated from a base station 6 by an arm 8.

The scanner system 3 is designed to move the focal spot F in the eye tissue 91, such that areas in the eye 9 designated for treatment are treated by the treatment laser beam T. The scanner system 3 may move the focal spot F by moving the optical system 2 in whole or in part. In other words, the optical system may be shifted laterally, e.g. in the x-y plane perpendicular to the z-direction such that all required areas of the eye tissue 91 are reachable by the treatment laser beam T. To achieve this, the scanner system 3 is coupled to the optical system 2 and comprises one or more actuators, such as linear piezo-electric actuators or electro-magnetic drives.

During treatment, the scanner system 3 moves according to a pre-defined scan pattern. More specifically, the ophthalmological laser treatment device 1 controls the scanner system 3 such that the image circle of the optical system 2 moves according to the pre-defined scan pattern.

The pre-defined scan pattern more specifically defines a so-called "slow" scan pattern, which governs or specifies the movement of the optical system 2 and thereby serves to move the image circle, and thereby also a central focal spot F, across the eye tissue 91. In other words, the scanner system 3 comprises a slow scanner, which may also be referred to as a main scanner, which is designed to move the treatment laser beam T according to the scan pattern.

The scanner system 3 may additionally include a so-called "fast" scanner which superimposes, on top of the "slow" scan pattern, an additional movement of the focal spot F. This additional movement is typically a movement lateral to a scan direction of the scan pattern, such that eye tissue 91 is treated in a band broader than the width of the focal spot F itself. In other words, the scanner system may also comprise a fast scanner, which may also be referred to as an auxiliary scanner, which is designed to move the treatment laser beam T, in particular the focal spot F, at a speed which is relatively speaking faster than the speed at which the slow scanner moves the focal spot F.

In particular, the fast scanner may move the focal spot F continuously or discretely along a line substantially perpendicular to the scan direction SD of the scan pattern. Preferably, the line extends across a substantial proportion (preferably at least a majority) of the image circle of the optical system 2.

The fast scanner may be implemented with a galvano scanner, acousto-optical scanner, electro-optical scan, a resonant scanner or a polygon scanner, which deflects the treatment laser beam T laterally. The fast scanner may be configured to deflect the laser beam laterally in one dimension only. The fast scanner may be configured to deflect the laser beam in two dimensions laterally.

For example, the slow scanner may move at least one optical element of the optical system such that the image area moves at a speed of between 10 mm/s and 100 mm/s. Equivalently, the slow scanner moves a center of the image area, which corresponds to a non-deflected position of the focal spot F (i.e. the position of the focal spot without the additional influence of the fast scanner). Thereby, the slow scanner moves the image area according to the scan pattern. The movement may be continuous or discrete, for example moving at a frequency of 100 Hz to 1 kHz, shifting 0.01 mm to 1 mm with each movement. The fast scanner may move at a relatively higher speed than the slow scanner, in particular at least an order of magnitude faster than the slow scanner. For example, the fast scanner moves or deflects the focal spot F from its central position in the image area along at least one axis within the image area. The fast scanner may deflect the focal spot F at a speed such that the focal spot F is deflected in the eye at a speed of 1000 mm/s - 10'000 mm/s. The fast scanner may be arranged upstream of the slow scanner. More specifically, the scanner system 3 may be designed such that the treatment laser beam T is first deflected by the fast scanner before passing the slow scanner.

The fast scanner and slow scanner may be co-located in the application head. The fast scanner and slow scanner may be arranged in separate locations, in particular being separated by the arm 8, for example with the fast scanner being arranged in the base station and the slow scanner arranged in the application head.

The ophthalmological laser treatment device 1 comprises an imaging system 4. The imaging system 4 may be arranged in close proximity to the optical system 2, for example arranged behind a beam splitter arranged upstream of the optical system 2 as shown in Fig. 2. The imaging system 4 may be arranged distant from the optical system, as shown in Fig. 3.

The imaging system 4 may be arranged upstream of the scanner system 3. The imaging system 4 may alternatively be arranged upstream of the slow scanner and downstream of the fast scanner.

The imaging system 4 comprises an image sensor 41. The image sensor 41 is arranged, depending on the embodiment, in the base station 6 or the application head 7. The image sensor 41 may comprise a photodetector array, for example a one-dimensional, two-dimensional, or three-dimensional array of photodetectors. The photodetector array is implemented, depending on the embodiment, as a CCD sensor or a CMOS sensor, for example.

The image sensor 41 is configured, depending on the embodiment, to be sensitive to one or more wavelengths (this includes a wavelength band) of light.

Specifically, the image sensor 41 is configured to generate a photodetector array signal depending on a light signal LS received by the image sensor 41. The light signal LS is comprised generally of light scattered in the eye tissue 91 by the treatment laser beam T.

In an embodiment, the imaging system 4 further comprises one or more filters arranged in front of the image sensor 41. The filters are configured to absorb, scatter, convert and/or reflect one or more wavelengths of light.

The filters comprise, for example, one or more colour filters, one or more spectral filters, one or more neutral density filters, one or more bandpass filters, one or more notch filters, one or more edge filters, one or more beamsplitter filters, one or more dichroic filters, one or more colour substrate filters, one or more excitation filters, one or more emission filters, and/or one or more polarization filters (e.g., linear and/or circular polarizers). The filters can cover the entire image sensor 41 or parts thereof, including individual photodetectors (e.g., a Bayer colour filter).

The image sensor 41 is configured to record one or more images. Preferably, the image sensor 41 is configured and/or controlled to record the one or more images with an exposure time of between 1/50th of a second to 1/10000ths of a second, most preferably in the range of 1/100ths to 1/1000ths of a second.

The image sensor 41 may be configured and/or controlled to record the one or more images at a defined frame rate of between 20 Hz and 1000 Hz, preferably 50 Hz to 200 Hz, most preferably 100 Hz.

In an embodiment, the frame rate may, however, alternatively be dynamically scaled according to the movement speed of the focal spot F and/or the time-points at which images are recorded may be synchronized with positions of the optical system, resp. scanner system. For example, when the position of a scanner position is at a defined point, the imaging system may be triggered to record an image.

Thereby, a defined lateral distance between each successive image is achieved. Specifically, the defined lateral distance may be the distance between centers of successive images and may be defined such the lateral distance is less than the width of the image, resulting in a defined amount of overlap between successive images. This results in a particular (sub)-area of eye tissue 91 being imaged more than once which allows for tracking that particular (sub)-area of eye tissue across a time-span.

The ophthalmological laser treatment device 1 comprises an electronic circuit 5. The electronic circuit 5 is configured to control the ophthalmological laser treatment device 1. The electronic circuit 5 embodies a programmable device and comprises, for example, one or more processors 51, and one or more memory modules 52 having stored thereon program code, data, as well as programmed software modules for controlling the processors 51, and/or other programmable circuits or logic units included in the electronic circuit 5, such as ASICs (Application-Specific Integrated Circuits), GPUs (graphics processing units), FPGA (field programmable gate arrays), and/or TPUs (tensor processing units). The memory modules 52 comprise volatile and/or non-volatile storage media, for example random access memory and/or flash memory, respectively.

The electronic circuit 5 is connected to other components and modules of the ophthalmological laser treatment device 1 as disclosed herein, in particular the laser source 11, the optical system 2, the scanner system 3, and the imaging system 4. The connection is a wired and/or wireless connection configured to exchange control and/or measurement signals. The connection may be in the form of a data bus.

The electronic circuit 5, depending on the embodiment, further comprises a communication interface 53. The communication interface is configured for data communication with one or more external devices. Preferably, the communication interface comprises a network communications interface, for example an Ethernet interface, a WLAN interface, and/or a wireless radio network interface for wireless and/or wired data communication using one or more networks, comprising, for example, a local network such as a LAN (local area network), and/or the Internet.

The electronic circuit 5 performs one or more steps and/or functions as described herein, for example according to the program code stored in the one or more memory modules. Additionally, or alternatively, the program code can be wholly or partially stored in one or more auxiliary processing devices, for example a computer. The skilled person is aware that at least some of the steps and/or functions described herein as being performed on the processor of the ophthalmological laser treatment device 1 may be performed on one or more auxiliary processing devices connected to the ophthalmological laser treatment device 1 using the communication interface. The auxiliary processing devices can be co-located with the ophthalmological laser treatment device 1 or located remotely, for example on a remote server computer.

The skilled person is also aware that least some of the data associated with the program code (e.g., application data) or data associated with a particular patient (e.g., patient data, or a treatment model comprising the scan pattern) and described as being stored in the memory 52 of the ophthalmological laser treatment device 1 may be stored on one or more auxiliary storage devices connected to the ophthalmological laser treatment device 1 using the communication interface.

The electronic circuit 5 stores, in the one or more memory modules 53, a treatment model. The treatment model is designed for treating the eye 91 of a patient 9 using the ophthalmological laser treatment device 1. In particular, the treatment model comprises a scan pattern which may define a number of treatment points or treatment curves onto which the treatment laser beam T is directed.

The ophthalmological laser treatment device 1 optionally includes a user interface comprising, for example, one or more user input devices, such as a keyboard, and one or more output devices, such as a display. The user interface is configured to receive user inputs from an eye treatment professional, in particular based on, or in response to, information displayed to the eye treatment professional using the one or more output devices.

**Figure 4** shows schematically some parts of an ophthalmological laser treatment device 1 related to generating the treatment laser beam T and recording an image. Specifically, the laser source 11 is depicted as generating the treatment laser beam T, which is incident on a beam splitter 42 and then travels to the eye issue 91. The imaging system 4 comprises an image sensor 41 arranged behind the beam splitter 42. The image sensor 41 in particular receives and records the light signal LS, which comprises light scattered by the eye tissue 91. The light scattered by the eye tissue may originate in the treatment laser beam T and/or in one or more lights of the ophthalmological laser treatment device 1, for example integrated into the application head. The lights may be controlled by the ophthalmological laser treatment device. The light scattered may include ambient light, for example provided by one or more lights or lamps present in the treatment facility in which the ophthalmological laser treatment device 1 is arranged.

The imaging system may be implemented using a digital camera which comprises the image sensor, such as a CMOS image sensor. The camera is configured to capture light in the visible, near-infrared, and/or ultraviolet spectral ranges. The camera is in particular configured to capture a two-dimensional image of the image circle or part thereof of the optical system. The image is therefore a two-dimensional image of part of the lens of the eye included in the image circle. In such a variant, the image is in particular not related to a depth scan and is not based on interferometry.

The camera may be configured or controlled to record images continuously during treatment, in particular when the treatment laser beam T is activated and incident on or in the eye tissue. Thereby, the camera records a real time live-feed of the treatment. The treatment laser beam T does not need to be shut off during or prior to recording of the images.

As depicted in Fig. 4, the treatment laser beam T is incident on, respectively projected onto, the eye tissue 91 at the focal spot F, which lies within an area of the eye tissue 91 defined by the imaging circle of the optical system 2 (not shown). The focal spot F moves according to the scan pattern S in a scan direction SD. The scan pattern comprises multiple neighboring scan lines SL1, SL2 and SL3.

For the sake of simplicity, the optical system 2 and scanner system 3, shown and described with reference to Figures 1 and 2, are omitted.

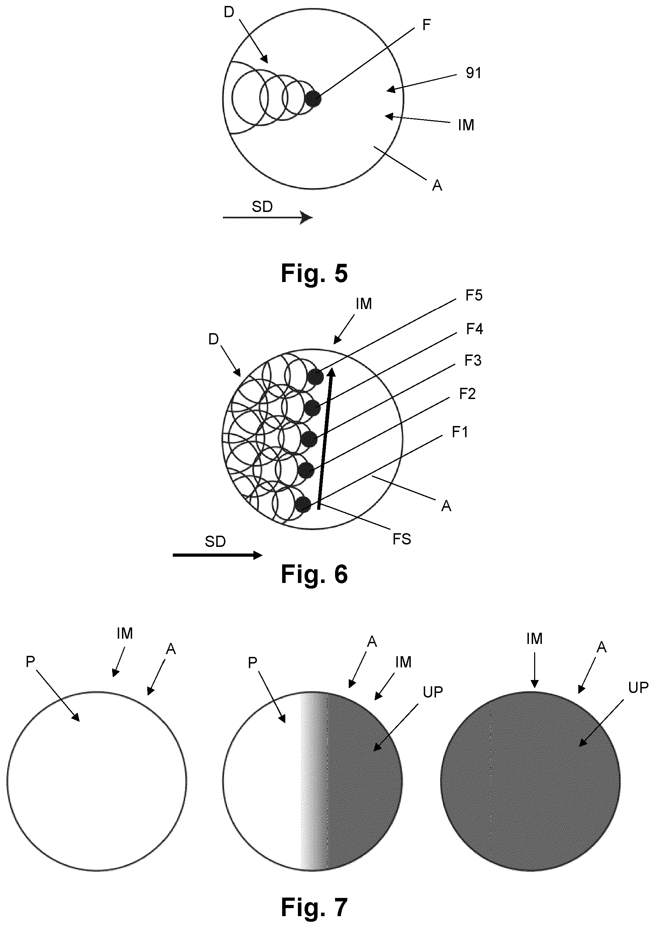

**Figure 5** shows a schematic illustration of an area A of the eye tissue 91. The area A corresponds to the image circle of the optical system and has a radius in a range between 0.1 mm and 3 mm, preferably between 0.5 mm and 2 mm, for example 1 mm. The focal spot F of the treatment laser beam is shown in the center of the area A. The treatment laser beam T modifies the eye tissue 91, for example by disrupting or ablating the eye tissue 91.

The focal spot F moves in the scan direction SD and, downstream from the focal spot F, i.e. in a direction opposite the scan direction SD, disrupted tissue D is illustrated by circles of increasing radius. This illustrates how the eye tissue 91 is modified not only at the focal spot F, but also, due to the energy deposited in the eye tissue 91 due to the treatment laser beam T, also eye tissue 91 in an area respectively volume surrounding the focal spot F is modified. This is, for example, due to the formation of gas bubbles in the eye tissue 91 which propagate outwards, modifying eye tissue 91 surrounding the focal spot F.

The image IM captured of the area A records, as is explained in more detail with reference to Figs. 6 to 8, the parts of the area A in which the eye tissue has been modified.

**Figure 6** shows an illustration of an area A of eye tissue 91 similar to Fig. 5, however showing multiple focal spots F1, F2, F3, F4, F5 arranged in a line in a fast scan direction FS. The fast scan direction FS forms an angle of substantially 90° to the (slow) scan direction SD. The multiple focal spots F1, F2, F3, F4, F5 are shown to be non-overlapping, however, depending on the implementation, the focal spots F1, F2, F3, F4, F5 may overlap. As illustrated, the treatment laser beam is targeted, by the fast scanner, to a plurality of focal spots F1, F2, F3, F4, F5 in sequence, resting at each focal spot F1, F2, F3, F4, F5 for a pre-defined duration and each focal spot F1, F2, F3, F4, F5 being arranged at a pre-defined position, resp. each focal spot F1, F2, F3, F4, F5 having a pre-defined distance relative to the other focal spots F1, F2, F3, F4, F5. The sequence of focal spots F1, F2, F3, F4, F5 results in a complete downstream tissue disruption, as illustrated by the overlapping circles of modified tissue D.

**Figure 7** shows three illustrations in sequence, illustrative of what the eye tissue 91, for example the cornea, would look like through the optical system at various stages of treatment, and thereby also illustrative of the images IM the imaging system records.

The left-most illustration shows processed eye tissue P, which is illustrated as having a white colour due to the fact that light is scattered by the eye tissue, in particular the cornea, after treatment due to ablation, disruption or more generally, tissue modification.

The middle illustration shows the eye tissue 91, in particular the cornea, during treatment, in which the eye tissue area viewable through the optical system is segregated or divided into two sections or halves, the left side having been modified and thereby being illustrated as processed eye tissue P, and the right side being illustrated as unprocessed eye tissue UP. The unprocessed eye tissue UP is darker than the processed eye tissue P because it is transparent to light and therefore the dark retina is visible through the eye tissue.

It is important to note that the division of the image IM into a left and right half, with the dividing line running vertically through the image, is due to the scan direction running from left to right. Scanning in other directions would naturally cause the image IM to be rotated accordingly.

The right-most illustration shows unprocessed eye tissue UP, i.e. eye tissue prior to treatment. An image of unprocessed eye tissue UP may be used as a baseline image, in particular for comparison with treated eye tissue, to determine the extent of modification of the eye tissue and therefore to serve as the basis for generating an eye tissue modification score.

In an embodiment where the images are mutually overlapping, each area of eye tissue may be imaged in more than one state of treatment, in particular prior to and subsequent to treatment. As such, each area of eye tissue is recorded both in an unmodified state and in a modified state. The image, resp. part of an image representing a particular area of eye tissue in a modified state may be subtracted, for example using a subtractive image processing filter, from another image, resp. part of another image representing the same particular area of eye tissue in an unmodified state.

The eye tissue modification score may thereby be generated, for the particular area, on the basis of a difference between the modified and unmodified state of the eye tissue as recorded in two or more images.

The eye tissue modification score is a value which indicates the extent of modification of the eye tissue. The value may be a binary, integer or floating-point value. The binary value may be defined to distinguish between unmodified and sufficiently modified eye tissue. The integer or floating-point values may be defined to indicate the degree of modification of eye tissue, for example using a defined scale. The value may indicate one of a plurality of discrete classes, each class indicative of a qualitative state of eye tissue, including, for example, unmodified, partially modified, and sufficiently modified.

The eye tissue modification score may be visually presented in a number of ways, for example using characters, symbols, textures, colours, icons, etc.

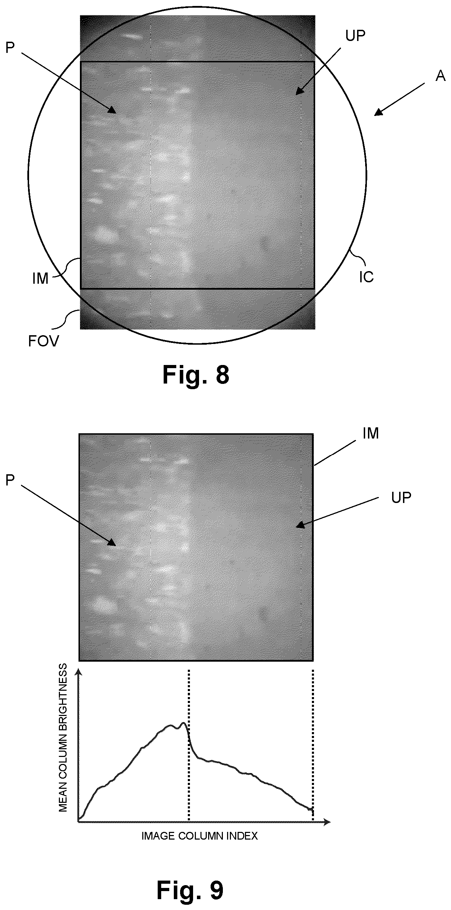

**Figure 8** shows an illustration including a recorded image IM of eye tissue during treatment of the eye. The image circle IC of the optical system is illustrated, which corresponds to the area A of the eye tissue viewable through the optical system. The field of view FOV of the image sensor is also depicted as a rectangular area which covers part of the image circle. The total field of view FOV may be cropped to remove any vignetting present to provide the (cropped) image IM.

As shown, the eye tissue, in particular the cornea, in the image IM is divided into two sections. The dividing line, which runs approximately in a straight fashion vertically through the middle of the image IM, is the line which corresponds to the fast scan direction of the treatment laser beam and comprises a plurality of focal spots.

The right section of the image IM includes unmodified or unprocessed eye tissue UP which has a largely uniform grey appearance, due to it being largely transparent and therefore the dark retina being visible through the cornea.

The left section of the image includes modified or processed eye tissue P which has a different appearance than the unprocessed eye tissue UP. In particular, it has a speckled appearance with bright spots and an overall lighter shade than the unprocessed eye tissue. This is due to the eye tissue having been modified, for example ablated or disrupted by the treatment laser beam. After modification, the eye tissue is no longer transparent but scatters light, causing a whiter appearance. The light scattered includes light originating from the treatment laser beam, as well as ambient light.

**Figure 9** shows an image IM recorded by an imaging system as described herein. The image IM shows eye tissue, in particular of the cornea, that is undergoing treatment. The left side of the image IM shows processed eye tissue P which has been processed by the treatment laser beam. The right side of the image IM shows unprocessed eye tissue UP which has not yet undergone treatment by the treatment laser beam.

The chart below the image IM shows the mean column brightness plotted against a column index of the image IM. The chart represents the average pixel brightness level of a plurality of vertical slices of the image IM. A dotted line is included, illustrating the division of the image IM into the left section of processed eye tissue P and the right section of unprocessed eye tissue UP.

As can be seen, there is a significant difference in the average column brightness between the left and right image halves. In particular, the right hand side shows a brightness level which steadily declines from the center of the image IM outwards. This is due to optical vignetting of the optical system.

The left-hand side shows a similar trend of the brightness level decreasing from the center outwards, however the peak average column brightness is significantly higher in the left hand side of the image IM than the right hand side.

As explained herein in more detail, the image IM can be automatically evaluated to generate an eye tissue modification score indicative of the extent of modification of the eye tissue. In particular, the difference between the left and the right side of the image IM can be quantized and/or otherwise used to determine whether the eye treatment was successful for a particular area A of the eye.

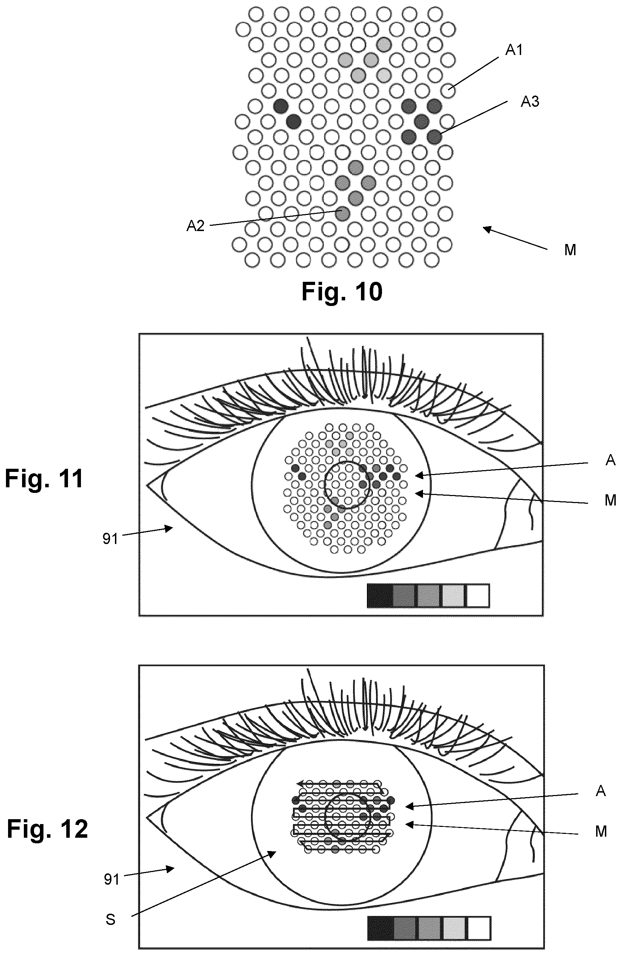

**Figure 10** shows a schematic illustration of an eye tissue modification map M which may be generated using a plurality of eye tissue modification scores generated for a plurality of positions in the eye. The eye tissue modification map M includes eye tissue modification scores graphically represented by circles shaded in different shades of grey tones, with white representing areas of eye tissue which has been successfully modified during treatment, and darker shades of grey representing areas of eye tissue only partially modified or not modified at all. Other colour schemes may of course be used.

In particular, the area A1 is shaded to indicate that the eye tissue has been modified correctly. The area A2 is shaded grey to indicate that the eye tissue has only partially been modified correctly, and may need subsequent treatment. The area A3 is shaded black, indicating that it has not been treated at all.

The eye tissue modification map M may be rendered or displayed in a variety of ways. In particular, the individual areas, each representative of the eye tissue modification score, may lie seamlessly next to each other. Additionally, each area A1, A2, A3 may also have a plurality of eye tissue modification scores calculated, for example one for each of a plurality of sub-areas.

**Figure 11** shows a schematic illustration of an eye tissue modification map M depicted on top of an image of an eye 9. An inserted legend indicates a plurality of different shades of grey to indicate a plurality of eye tissue modification scores visually. The image of the eye 9 may be a live image or image-feed, received from a camera, for example. The image of the eye 9 may be an image of the eye 9 recorded prior to treatment, for example. The image of the eye 9 may also be an image of a generic eye 9 (i.e., not of an eye 9 of the patient). The eye tissue modification map M is rendered together with the eye 9 such that eye tissue modification map M is positioned, scaled and oriented correctly. The eye tissue modification map M may be positioned, scaled and oriented (e.g., rotated) with respect to a coordinate system. In particular, the eye 9, respectively parts of the eye 9 such as the corners of the eye, the iris boundary, and/or the pupil, has defined coordinates in the coordinate system. The eye tissue modification map M or the corresponding treatment model also has defined coordinates in the coordinate system.

The eye tissue modification map M is then rendered correctly according to the coordinate system.

Depending on the treatment model, the eye tissue modification map M may comprise one or more layers, the layers corresponding to different depth regions in the eye 9 (depth being defined along the optical axis of the optical system or along the optical axis of the eye).

The eye tissue modification map M may be rendered such as to display not the whole eye tissue modification map M at the same time. For example, a single layer may be rendered and displayed at a time. The eye tissue modification map M may be rendered such as to display different layers side-by-side. In other words, different eye tissue modification maps M may be rendered for different layers and optionally displayed next to each other. In an embodiment, the different layers are rendered simultaneously in a three dimensional visualization.

**Figure 12** shows a variation of the schematic illustration of Fig. 11, further including the scan pattern S rendered as a path in the eye tissue modification map M. As depicted, the scan pattern S comprises the spots which lie along the path of the scan pattern. which comprise the eye tissue modification map M, each spot visually indicating the eye tissue modification score for that particular position in the eye. The scan pattern S is rendered such that its position, scale and/or orientation matches that of the eye tissue modification map M.

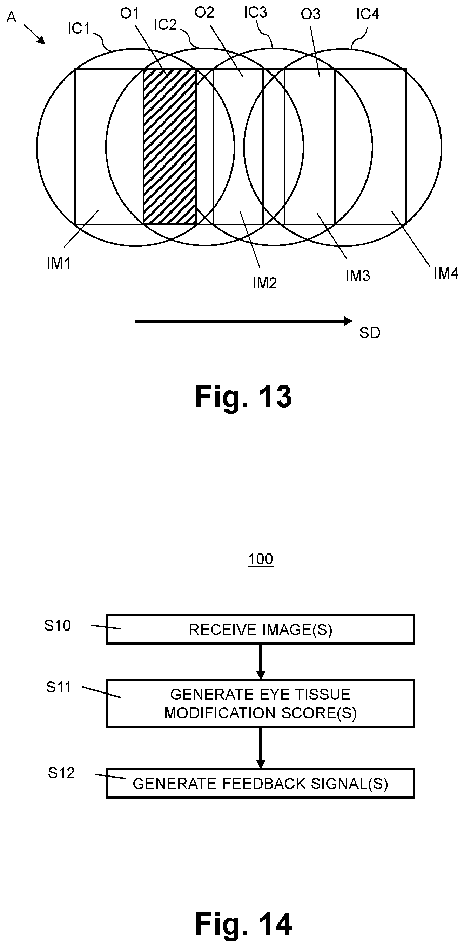

**Figure 13** shows a schematic illustration of a plurality of image circles IC1, IC2, IC3, IC4 at a plurality of positions in the eye tissue. The image circles IC1, IC2, IC3, IC4 lie in a line in scan direction SD.

The image circles IC1, IC2, IC3, IC4 and thereby also the areas of eye tissue they cover, overlap. The image circles IC1, IC2, IC3, IC4 are shown at particular discrete positions which are associated with time-points where the imaging system recorded the images IM1, IM2, IM3, IM4. The images IM1, IM2, IM3, IM4 may be cropped images. In other words, the raw image sensor field of view may extend beyond the boundaries of the image circles IC1, IC2, IC3, IC4.

As shown in the figure, the images IM1, IM2, IM3, IM4 partially overlap. In particular, images IM1 and IM2 overlap in an overlap area O1 as indicated by the hashing. Likewise, images IM2 and IM3 overlap in an overlap area O2, and images IM3 and IM4 overlap in an overlap area O3.

Depending on the implementation, the size of the overlap areas O1, O2, O3 may be between 25% and 90% of the area of each image IM1, IM2, IM3, IM4. Preferably, the size of the overlap areas O1, O2, O3 are at least 50% of the area of each image IM1, IM2, IM3, IM4. In other words, the center to center distance between two adjacent images IM1, IM2, IM3, IM4 is preferably half the width of each image IM1, IM2, IM3, IM4. By having at least 50% overlap, the modified eye tissue, i.e. the eye tissue in its state after treatment, can be completely captured in the images IM1, IM2, IM3, IM4. This is because, for any given image recorded during treatment, only half the image IM1, IM2, IM3, IM4 is of processed eye tissue, while the other half is of unprocessed eye tissue (i.e., eye tissue which the treatment laser beam has not yet modified).

Further, the more overlap there is between individual images IM1, IM2, IM3, IM4, the more data is available to subsequently analyze the eye tissue and generate the eye tissue modification score.

The overlapping images IM1, IM2, IM3, IM4 or parts thereof may be at least partially stacked and/or merged to improve a signal to noise ratio for imaged eye tissue. Alternatively, separate eye tissue modification scores may be calculated for each image IM1, IM2, IM3, IM4 as described herein to resolve the eye tissue or parts thereof at a greater resolution than available from a single image.

**Figure 14** shows a flow diagram illustrating a method 100. The method 100 may be performed by an electronic circuit 5 of an ophthalmological laser treatment device. The method 100 may alternatively be performed by an external or auxiliary electronic circuit, such as a processor of a computer.

The method 100 may be performed during or after treatment. The method 100 may, for example, be performed in a recurrent fashion, preferably in real-time or in near real-time, during treatment.

The method 100 comprises a plurality of steps S10 - S12.

In step S10, the electronic circuit receives one or more images from the imaging system. The images may be stored in the memory 52.

In particular and as described herein, the imaging system captures a sequence of images as the optical system and scanner system traverse the treatment area. Each image is associated with positional data relating to the position of the image, which may be derived from the scan pattern, time-stamps, or direct feedback from the scanner system, thereby enabling precise mapping of each image to a corresponding location or position on the eye.

The images may be cropped, for example such that the (cropped) image is constrained to the image circle of the optical system.

The images may be pre-processed, for example by being converted to gray-scale, resized/rescaled.

The images may be rotated such that a dividing line between processed and unprocessed eye tissue runs vertically through the image.

The images may be further cropped such that the image represents only modified eye tissue. In particular, parts of, or all of the side of the image representing unprocessed tissue may be cropped away. The resulting cropped images may then be merged, stitched, or otherwise assembled together with one or more adjacent (neighboring) images in an overlapping and/or non-overlapping manner, according to the distance between the images in the eye tissue.

Overlapping areas may be merged using averaging, blending, or selection of the highest-quality data, thereby reducing artifacts and improving the continuity of the image data.

The distance between adjacent images may be less than or equal to half the width of each image. More generally, the distance between adjacent images in the scan direction may be less than or equal to half the diameter of the images in scan direction.

Thereby, a mosaic image generated using one or more images of modified eye tissue may be generated. The mosaic image of modified eye tissue may be used for further processing according to one of the methods herein.The received images may be associated with the one or more positions in the eye. For example, information may be received in the electronic circuit indicative of the position of the focal spot and/or the imaged area of eye tissue. This information may relate to a position and/or orientation of the optical system, a position of the scanner system, and/or a position of the application head.

The information may comprise the scan pattern of the treatment model. For example, the scan pattern may define one or more positions in the eye that will be or have been targeted for treatment.

The electronic circuit may associate the received images with the one or more positions in the eye further using one or more defined time-points related to the treatment of the eye, for example a treatment start time point, and one or more time-stamps associated with the one or more received images, respectively.

In particular, the images or image data generated by processing the images are assembled into a single composite image, the mosaic image, by placing each image at its corresponding position within a reference coordinate system. The reference coordinate system may be defined relative to anatomical features of the eye or the scan pattern employed during treatment as described herein, for example. Areas of the eye not yet treated, or areas between non-overlapping images may be rendered as transparent or white, for example.

The mosaic image may be updated in real time or near real time as new images are acquired during treatment. This enables the mosaic image to reflect the current status of the eye tissue modification process, providing a live overview to the operator.

By amalgamating multiple partial images into a single, large-area representation, the mosaic image enables visualization of the entire treated region of the eye, overcoming the limitations of the restricted field of view inherent to the optical system. The mosaic image allows an operator to quickly visually assess the extent and uniformity of tissue modification across the treatment area. Regions of insufficient or incomplete modification can be readily identified, as can areas where treatment has been successfully completed. Optionally, the extend and uniformity of the tissue modification can be assessed in an automated manner, for example by generating an eye tissue modification score as detailed below.

The mosaic image may in particular be used to generate or update an eye tissue modification map, which can be overlaid on anatomical images (and/or the mosaic image) of the eye. This facilitates targeted post-processing or retreatment of areas identified as insufficiently modified, thereby improving treatment outcomes.

The mosaic image can be displayed on a user interface, optionally with overlays indicating a scan patterns, eye tissue modification scores, or anatomical landmarks such as the pupil, iris features, etc. This enhances the operator's situational awareness and supports informed decision-making during and after the procedure.

The mosaic image may further be stored or recorded to memory and provides a record of the treatment process, providing evidence of the areas treated and the degree of tissue modification achieved.

The further steps S11 and S12 are optional and may be performed to generate an eye tissue modification score for one or more locations in the mosaic image.

In step S11, an eye tissue modification score is generated. The eye tissue modification score is generated using the one or more images received in step S10, which images may have been subject to pre-processing (e.g., cropping or merging). The eye tissue modification score is designed to indicate or be representative of an extent of modification of the eye tissue caused by the treatment laser beam. The eye tissue modification score may therefore be used as a measure of how successful the eye tissue modification was, specifically for the eye tissue represented in the image(s).

The eye tissue modification score may be generated using one or more image analysis algorithms and several different approaches are known which may be used to implement the generation of the eye tissue modification score.

The eye tissue modification score may be a binary score, indicating whether the eye tissue has been successfully modified according to the treatment model, or not successfully modified. The eye tissue modification score may, alternatively or additionally, be indicative of the degree to which the eye tissue was modified successfully. The eye tissue modification score may be qualitative and/or quantitative in nature.

In step S12, one or more feedback signals or messages are generated. The feedback signals depend on the eye tissue modification score. The feedback signals, for example, may be sent only if the eye tissue modification score has a particular value and/or satisfies one or more defined thresholds. The feedback signals may comprise, reference and/or be indicative of the eye tissue modification score.

The feedback signals may further include or reference the image(s) used to generate the eye tissue modification score, one or more time-stamps indicative of the time-points that the one or more images were taken, an indicator or reference to the scan pattern, in particular one or more treatment points of the scan pattern associated with the images, and/or an indicator or reference to spots, points and/or locations in the eye tissue with which the one or more images used to generate the eye tissue modification score are associated. In other words, the feedback signal(s) may comprise, besides the eye tissue modification score, further information enabling for mapping the eye tissue modification score to a particular part of the eye tissue, allowing for traceability such that, in a possible subsequent step, those parts of the eye tissue insufficiently modified may undergo further (post) treatment.

The feedback signal(s) may be stored to the memory. The feedback signals may be provided to an output device, such as a display, of the ophthalmological laser treatment device or connected to ophthalmological laser treatment device. The feedback signals may be transmitted to an external device for storage and/or further processing.

According to the feedback signal, the operator of the ophthalmological laser treatment device may therefore continue the current treatment as planned, stop the current treatment, adjust the operating parameters of the ophthalmological laser treatment device (e.g., beam power), or re-treat particular areas identified as not having been (sufficiently) modified.

In an optional further step, the ophthalmological laser treatment device is configured to generate a post-processing treatment model to treat points and/or areas of eye tissue not sufficiently modified, using the one or more points or areas of eye tissue which were not modified.

The post-processing treatment model defines a post-processing scan pattern including the one or more points or areas of eye tissue which were not disrupted and optionally includes customized beam parameters for each of the points or areas of eye tissue depending on the eye tissue modification score.

In an optional further step, treatment parameters of the ophthalmological laser treatment device are adjusted according to the eye tissue modification score. The treatment parameters include the scan pattern, a beam power, a repetition rate of the treatment laser beam, fast scan parameters relating to the distance between fast scan spots and/or a duration of applying the treatment laser beam to each fast scan spot, and/or a focal spot size.

For example, the beam power may be increased if the eye tissue modification score is indicative of eye tissue having not been modified. Preferably, the beam power is adjusted as a function of the eye tissue modification score.

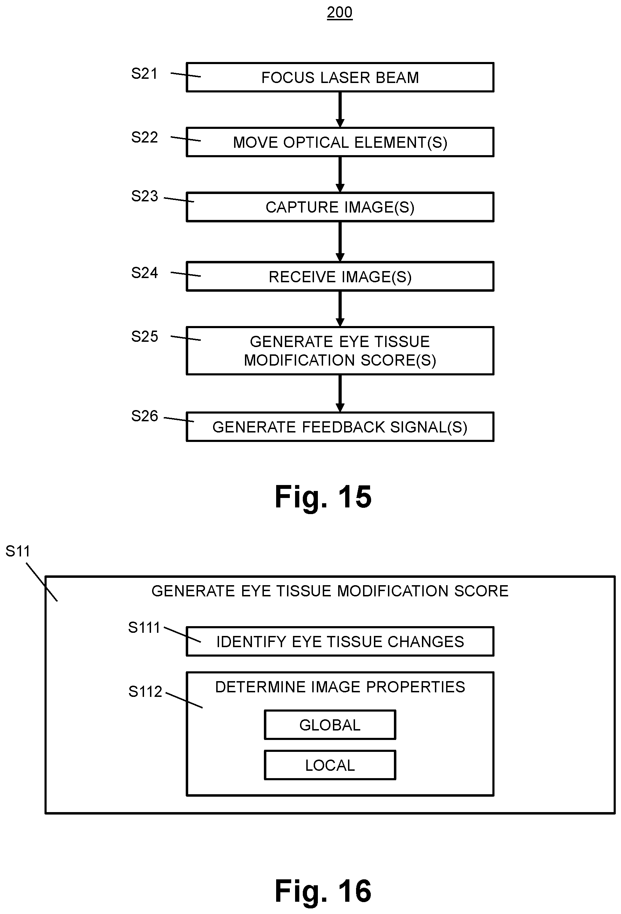

**Figure 15** shows a flow diagram illustrating a method 200. The method 200 is performed by the ophthalmological laser treatment device. The method 200 is in particular performed by the electronic circuit in conjunction with other components. The method 200 is performed during treatment of an eye of a patient. The method 200 comprises steps S21 to S26. Steps S24 to S26 correspond to steps S10 to S12 of method 100.

In step S21, the treatment laser beam is focused by the optical system onto a particular point in the eye tissue according to the scan pattern.

In step S22, the focal spot is moved in the eye tissue, which may comprise a lateral movement in the x-y plane as well as a movement in the depth (z) direction, in scan direction according to the scan pattern. The focal spot is moved by moving, using the scanner system, at least one of the optical elements.

In step S23, one or more images of an area of eye tissue surrounding the focal spot are captured by the image sensor of the imaging system. The images may be captured at a defined frame rate, for example at 100 Hz.

In an embodiment, the movement of the focal spot and the capturing of the one or more images is coupled, for example such that images are captured at a defined spatial frequency (e.g., one image for every millimeter the focal spot moves in scan direction).

**Figure 16** shows a diagram illustrating a number of steps, one or more of which may be performed for generating the eye tissue modification score.

In the optional step S111, eye tissue changes are identified in the one or more images, in particular in the left hand side of the one or more images. These eye tissue changes relate to structural changes in the eye tissue due to the treatment laser beam depositing energy in the eye tissue at and around the focal spot.

The changes relate to one of more of: gas created inside the modified eye tissue, a change in a refractive index of the modified eye tissue, a change in the polarization of light in the eye tissue due to it having been modified, and/or a change in the color of the eye tissue due to it having been modified.

These one or more changes are identified by the electronic circuit and may be used in the methods described herein to generate the eye tissue modification score.

In particular, the gas bubbles may be identified in a region of the image counter to a scan direction of the focal spot/optical system. The gas bubbles may be identified as bright spots or regions in the part of the image representing processed eye tissue.

The scan direction may be received in the electronic circuit from the scanner, from an optical system and/or be determined using the scan pattern.

The scan direction may also be determined using the image alone, for example by detecting a line or band of division between the processed and unprocessed parts (roughly halves) of the image.

In addition or alternatively, a current and optionally one or more previous laser spots in the eye tissue may be identified, the laser spots resulting from the treatment laser impinging on the eye tissue at the focal spot.

In optional step S12, image properties are determined for the image to generate the eye tissue modification score. The image properties may include global image properties and/or local image properties.

The global image properties may include, for example, an overall brightness distribution (e.g., a contrast level) in the image, the contrast level determined by comparing the brightness level between two or more pixels, or two or more sets of pixels. For example, a set of pixels may define a column in the image, the columns arranged in parallel direction to the dividing line between the processed and unprocessed regions of eye tissue represented in the image.

Other global image properties such as colors may also be used. The global image properties of the received images may be compared to baseline global properties of one or more baseline images recorded prior to treatment. If the global image properties are significantly different to the baseline image(s), then it may be positively determined that the eye tissue has been disrupted. The degree of difference with respect to the baseline images may be quantified in the eye tissue modification score. The eye tissue modification score may, additionally or alternatively, indicate whether the degree of difference is larger than a defined threshold, thereby indicating sufficient modification for successful treatment of the eye tissue.

The local image properties include, for example, a local or micro-contrast level in one or more particular parts of the image. The parts of the image may be defined as two dimensional areas. The parts of the image may be, for example, defined as overlapping or non-overlapping squares of 32 x 32 pixels, or 16 x 16 pixels. The parts of the image may be defined as lines, for example columns and/or rows spanning the height and/or width of the image and having a defined width of e.g., 8 pixels or 16 pixels. These lines may be arranged parallel to the scan direction.

Local image properties include, for example, a contrast or micro contrast level of one or more parts of the image, a dynamic range of one or more parts of the image, a level of structuring of one or more parts of the image, and/or a colour of one or more parts of the image.

The level of structuring is given by textures, patterns, lines, features, and/or color variations of the eye tissue. The aforementioned are due to visible physical alternations of the eye tissue due to the treatment laser beam. The level of structuring of the eye tissue depends on the extent of modification of the eye tissue due to the treatment.

In one example, the one or more images may be divided into two sections by a line which intersects the focal spot at an angle to the scan direction (in particular, the angle at which the treatment laser beam is moved by a fast scanner of the scanner). The average brightness of the two sections may be compared to generate the eye tissue modification score.

The eye tissue modification score may be generated using the global image properties and/or the local image properties. For example, greater levels of contrast and/or greater differences to a baseline image may result in a higher eye tissue modification score.

The eye tissue modification score may be further generated using one or more image processing filters. The image processing filters may process the image by selecting, removing, and/or modifying particular parts or aspects of the image and may be configured to generate, using the processed image, the eye tissue modification score. The image processing filters may comprise one or more image convolution filters in which a kernel or mask is applied to the image.

The image processing filters may comprise a neural network, in particular a convolutional neural network comprising one or more convolutional layers and trained on image processing.

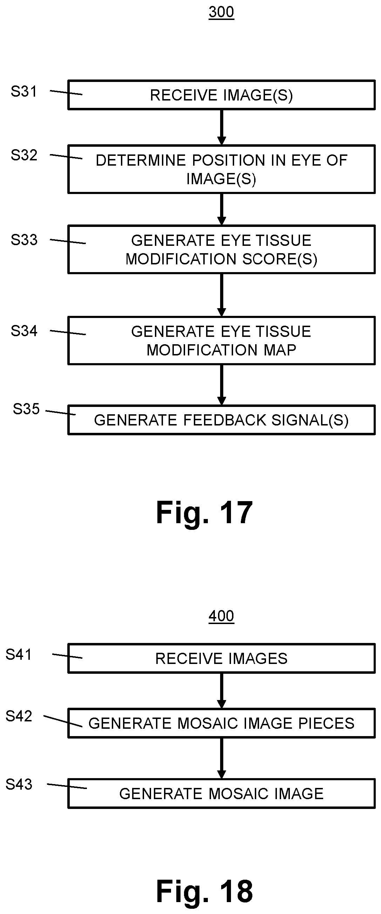

**Figure 17** shows a flow diagram illustrating a method 300 for generating an eye tissue modification map. The method 300 may be performed by the electronic circuit of the ophthalmological laser treatment device. The method 300 may be performed by an electronic circuit external to the ophthalmological laser treatment device.

The method 300 may be performed recurrently, in particular during treatment of an eye. The method 300 may, additionally or alternatively, be performed subsequent to treatment, and/or prior to further treatment.

The method 300 comprises a number of step S31 to S35. Some of these steps are analogous to steps described herein with reference to the methods 100 and/or 200.

In step S31, which is analogous to step S10 of method 100, one or more images of the eye tissue are received. The images may be received from the imaging system or from memory.

In step S32, one or more positions in the eye are determined for the one or more received images, respectively. In other words, each image has associated with it a particular position in the eye. An indicator of the position, for a particular image, may be included with the image itself, for example in meta-data of the image. Other methods for determining the position of the image are also possible, for example the position may be determined by comparing a time-stamp of the image with a scan pattern, the scan pattern defining, each position in the eye to be treated, an associated time-point at which that position will be treated.

The scanner system and the imaging system may be synchronized such that images are recorded by the imaging system at time-points where the scanner system is in defined positions. The synchronization may be coordinated by the electronic circuit, for example by transmitting trigger signals to the scanner system and/or the imaging system.

In step S33, eye tissue modification scores are generated. The eye tissue modification scores are generated using the one or more images. Each eye tissue modification score is implicitly or explicitly linked or associated with a particular position in the eye.

An eye tissue modification score may be generated for each image individually as described herein, the eye tissue modification score for a particular image being associated with the position of the particular image.

Additionally or alternatively, two or more of the images may be combined to form a mosaic image as described herein, the position of a particular image in the mosaic image being determined by the position in the eye associated with the particular image. More specifically, the part of each image representing processed, resp. modified eye tissue is used when generating the mosaic image. The mosaic image is preferably generated using only those parts of the captured images which represent processed eye tissue.

A plurality of eye tissue modification scores may then be generated using the mosaic image. Specifically, the mosaic image may be divided in to a plurality of segments (for example, square segments), which segments may have an area larger than or smaller than the area represented by any one of the originally captured images. Each segment is generated at a defined position in the mosaic image, and therefore each segment also has a therewith associated position in the eye.

The segments, which may be square segments, circular segments, or otherwise shaped segments, may be represented by a group of one or more pixels. The pixels in a particular segment are generated using the images associated with the particular segment (for example by having a position in the eye corresponding to the position of the particular segment) and representing visually the processed tissue recorded in those images.

The mapping between images and mosaic segments may be 1:1, such that each image corresponds to one particular mosaic segment and vice versa. However, in general, one or more images may be used to generate one or more mosaic segments.

The mosaic image therefore provides an amalgamated image of the eye, in particular the area of the eye being treated or having been treated. For each segment of the mosaic image, an eye tissue modification score may be generated according to one of the methods described herein. For example, each segment may be compared to a baseline image representing unprocessed eye tissue.

In step S34, an eye tissue modification map is generated. The eye tissue modification map comprises a plurality of eye tissue modification scores arranged according to their position relative to each other. Each eye tissue modification score may be represented using a visual encoding scheme, for example mapping eye tissue modification scores to colours, shades of grey, patterns, icons and/or other symbols.

The eye tissue modification map, respectively each individual eye tissue modification score, may be rendered in two dimensions, for example as depicted in Fig. 10, where the eye tissue modification scores are discretized into a plurality of shades of grey representing different degrees or extents of eye tissue modification.

The eye tissue modification map may further be rendered on top of an image of an eye as depicted in Fig. 11. The image of an eye may be a generic image of an eye, or a specific image of an eye of the patient previously recorded.

In an embodiment, the eye tissue modification map may be updated during treatment. In particular, images newly received from the imaging system may be used to update the eye tissue modification map, for example by adding newly eye tissue modification scores generated based on the newly received images.

In step S35 the feedback signal is generated to include the eye tissue modification map. The eye tissue modification map may be displayed on a display, for example a display connected to the ophthalmological laser treatment device. Thereby, an operator of the ophthalmological laser treatment device may refer to the eye tissue modification map during or after treatment to assess whether the eye tissue was modified according to the treatment model, whether the treatment was successful, whether further treatment is necessary or not, etc. Thereby, the quality of the eye treatment may be improved.

**Figure 18** shows a flow diagram illustrating a method 400 for generating a mosaic image. The mosaic image is of the eye tissue, in particular comprising a reproduction or representation of modified (processed) eye tissue. The method 400 may be performed by the electronic circuit of the ophthalmological laser treatment device. The method 400 may be performed by an electronic circuit external to the ophthalmological laser treatment device. The method 400 may be performed as an alternative to, or in addition to, one or more of the other methods described herein. The method 400 comprises a number of step S41 - S43. The steps S41 - S43 may be performed in sequence. At least some of the steps S41 - S43 may be performed recurrently.

In step S41, a plurality of images are received. The images are of eye tissue, in particular as recorded by an imaging system of an ophthalmological laser treatment device as described herein. The images are partial views of the eye tissue in that the images do not cover the entire eye, more particularly, do not cover the entire eye tissue which is being or was treated. In an embodiment, each image includes an area representing modified or processed eye tissue and an area representing unmodified or unprocessed eye tissue.

The images may be received directly from the imaging system. The images may alternatively or additionally be received from an intermediary system, which may comprise a memory or other data store.

The images may be overlapping, in other words, a given image may represent an area of eye tissue which is also (at least partially) represented in one or more other images. Thereby, a particular area of eye tissue may be recorded in a plurality of images.

In step S42, mosaic image pieces are generated using the plurality of received images. For example, each image is cropped such as to generate a mosaic image piece which does not overlap with a neighboring mosaic image piece. In another example, two or more images are used to generate a single mosaic image piece.

More generally, the mosaic image pieces are generated using the plurality of received images and image processing. For example, several images may be used to generate a single mosaic image piece. In another example, a single image may be used to generate several mosaic image pieces.

As described herein, each image may be cropped such as to include only areas of processed eye tissue (or equivalently, unprocessed areas of eye tissue in each image are cropped away). The cropped images may be considered individual mosaic image pieces.

As described herein, the images may be individually and/or collectively processed, using image processing comprising steps such as merging, splitting, rotating, filtering, or otherwise transforming such as to generate mosaic image pieces which can be used to generate a mosaic image.

For example, the mosaic image pieces may indicate or represent a difference between the modified tissue and unmodified tissue. This may be achieved by subtracting the image, in particular those parts of the image which represent modified tissue, from a part of the image which represents unmodified tissue. The subtractive filter may work on a pixel by pixel basis.

In an embodiment, the mosaic image pieces each have a defined position. The defined position may be determined using the positions of the images as described herein, in particular the position is defined relative to the scan pattern. The position of each mosaic image piece is defined such that the individual mosaic images can be assembled to form a collective mosaic image which covers at least part of the eye tissue which was, or is being, treated.

For example, pixel coordinates of pixels a particular image, along with the position of the particular image, can be used to generate, for a mosaic image piece generated using that particular image, the defined position of the mosaic image piece.

For purposes of defining the position of the mosaic image pieces, a reference coordinate system may be used or established, which may be based on the treatment model and/or scan pattern, or additionally or alternatively, defined coordinates or positions in the eye.

Steps S41 and S42 may be performed recurrently as new images are received, for example images newly captured by the imaging system.

In step S43, the mosaic image is generated. The mosaic image is generated by assembling the mosaic image pieces according to their defined position. The resulting mosaic image represents the eye tissue, in particular the eye tissue which has been treated and imaged.

The mosaic image may be continually updated using newly generated mosaic image pieces. Thereby, during treatment, the mosaic image continually represents a current real-time or near-real time state of the eye undergoing treatment.

The mosaic image may be transmitted as part of the feedback signal(s). The mosaic image may be displayed, on a display, to an operator of the ophthalmological laser treatment device. The mosaic image may be displayed alone or in addition to the eye tissue modification map.

One or more eye tissue modification scores may be generated using the mosaic image. For example, an eye tissue modification score may be generated for each mosaic image piece, each mosaic image piece representing a defined area of the eye tissue.

In an embodiment, an aggregate eye tissue modification score is generated using one or more eye tissue modification scores. The aggregate eye tissue modification score is in particular generated using a defined function which receives as an input the one or more eye tissue modification scores. The aggregate eye tissue modification score is a single score which defines the extent to which the eye treatment was successful.

The above-described embodiments of the disclosure are exemplary and the person skilled in the art knows that at least some of the modules and/or steps described in the embodiments above may be rearranged, omitted, or introduced into other embodiments without deviating from the scope of the present disclosure.

## Claims

1. An ophthalmological laser treatment device (1), the ophthalmological laser treatment device comprising:
an optical system (2) comprising a plurality of optical elements (21) which optical system (2) defines an image area and is configured to focus a treatment laser beam (T) onto a focal spot (F) in eye tissue (91) of an eye (9);
a scanner system (3) coupled to the optical system (2) and configured to move at least one of the optical elements (21) such that the focal spot (F) in the eye tissue (91) moves according to a scan pattern, wherein the scanner system (3) comprises a slow scanner configured to move the image area and thereby also the focal spot (F) according to the scan pattern and a fast scanner which superimposes, onto the scan pattern, an additional movement of the focal spot (F) at a speed which is faster than the speed at which the slow scanner moves the focal spot (F);
an imaging system (4) optically coupled to the optical system (2) and configured to capture one or more images (IM) of the area (A) surrounding the focal spot (F) located within the image area; and
an electronic circuit (5) configured to:
receive (S1), from the imaging system (4), the one or more images (IM),
generate (S2) an eye tissue modification score, using the one or more images (IM), the eye tissue modification score being indicative of an extent of modification of the eye tissue (91) achieved by moving the focal spot (F) of the treatment laser beam (T) in the eye tissue (91), and
generate (S3) one or more feedback signals depending on the eye tissue modification score.

2. The ophthalmological laser treatment device (1) according to claim 1, wherein the electronic circuit (5) is further configured to identify eye tissue changes in the one or more images (IM) due to photo modification of the eye tissue by the treatment laser beam (T), the changes relating to one of more of: gas created inside the eye tissue (91), a change in a refractive index of the eye tissue (91), a change in the polarization of light due to the eye tissue, or a change in the color of the eye tissue (91), and to generate the eye tissue modification score using the identified eye tissue changes.

3. The ophthalmological laser treatment device (1) according to one of claims 1 or 2, wherein the imaging system (4) is configured to capture the one or more images (IM) during activation of the treatment laser (T), and the electronic circuit (5) is configured to generate (S2) the eye tissue modification score using the one or more images (IM) captured during activation of the treatment laser (T), such that the eye tissue modification score is indicative of the dynamic process of eye tissue modification.

4. The ophthalmological laser treatment device (1) according to one of claims 1 to 3, wherein the imaging system (4) is configured to capture one or more two-dimensional images (IM) of the eye tissue (91) formed in an image plane of the optical system (2).

5. The ophthalmological laser treatment device (1) according to one of claims 1 to 4, wherein the electronic circuit (5) is configured to determine global image properties in the one or more images (IM) and to generate the eye tissue modification score depending on the global image properties.

6. The ophthalmological laser treatment device (1) according to one of claims 1 to 5, wherein the image processing comprises determining local image properties in the one or more images (IM).

7. The ophthalmological laser treatment device (1) according to one of claims 1 to 6, wherein the imaging system (4) is optically coupled to the optical system by way of a beam splitter (41) such as to capture the one or more images (IM) of the eye tissue (91) coaxially through the optical system (2).

8. The ophthalmological laser treatment device (1) according to one of claims 1 to 7, wherein the imaging system (4) captures a plurality of images (IM1, IM2, IM3, IM4) in which at least some adjacent images (IM1, IM2, IM3, IM4) partially overlap each other in image overlap regions (O1, O2, O3), and wherein the eye tissue modification score is determined for a particular image (IM2) or a particular area of eye tissue captured by the particular image (IM2) using image data from the one or more image overlap regions (O1, O2) of the further images (IM1, IM3) adjacent to the particular image (IM2).

9. The ophthalmological laser treatment device (1) according to claim 8, wherein at least some of the adjacent images (IM1, IM2, IM3, IM4) comprise image overlap regions (O1, O2, O3) of eye tissue (91), wherein the adjacent images (IM1, IM2, IM3, IM4) overlap each other by at least 90%, preferably at least 95%.

10. The ophthalmological laser treatment device (1) according to one of claims 1 to 9, wherein the electronic circuit (5) is further configured to associate the one or more received images (IM) with one or more positions in the eye (9), respectively.

11. The ophthalmological laser treatment device (1) according to claim 10, wherein the electronic circuit (5) is further configured to generate an eye tissue modification map (M) using the eye tissue modification score(s), the eye tissue modification map (M) comprising, for each of a plurality of positions in the eye (9), an indicator of whether the eye tissue (91) has been modified.

12. The ophthalmological laser treatment device (1) according to claim 11, wherein the electronic circuit (5) is further configured to provide, to a display, an image of the eye (9) including an overlaid rendering of the eye tissue modification map (M).

13. The ophthalmological laser treatment device (1) according to one of claims 1 to 12, wherein the feedback signal is indicative of eye tissue (91) which, after having been targeted by the focal spot (F), was not modified.

14. The ophthalmological laser treatment device (1) according to one of claims 1 to 13, wherein the electronic circuit (5) is further configured to adjust treatment parameters of the ophthalmological laser treatment device (1) depending on the eye tissue modification score, the treatment parameters including one or more of: the scan pattern, a beam power, a repetition rate of the treatment laser beam, or a focal spot size.

15. The ophthalmological laser treatment device (1) according to one of claims 1 to 14, wherein the electronic circuit (5) is further configured to generate a mosaic image using the one or more images (IM) and to generate, for a plurality of positions in the mosaic image, the eye tissue modification score.

16. A method for operating an ophthalmological laser treatment device (1) comprising: an optical system (2) including a plurality of optical elements (21) which optical system (2) defines an image area, a scanner system (3) coupled to the optical system (2), wherein the scanner system (3) comprises a slow scanner and a fast scanner, an imaging system (4), and an electronic circuit (5), wherein the electronic circuit (5) is configured to perform the steps of:
focusing (S21), using the optical system (2), a treatment laser beam (T) onto a focal spot (F) in eye tissue (91) of an eye (9);
moving (S22), using the scanner system (3), at least one of the optical elements (21) such that the focal spot (F) in the eye tissue (91) moves according to a scan pattern (S), wherein the slow scanner moves the image area and thereby also the focal spot (F) according to the scan pattern and the fast scanner superimposes, onto the scan pattern, an additional movement of the focal spot (F) at a speed which is faster than the speed at which the slow scanner moves the focal spot (F);
capturing (S23), using the imaging system (4), one or more images (IM) of an area (A) surrounding the focal spot (F) located within the image area;
receiving (S24), from the imaging system (4), the one or more images (IM);
generating (S25) an eye tissue modification score, using the one or more images (IM), the eye tissue modification score being indicative of an extent of modification of the eye tissue (91) achieved by moving the focal spot (F) of the treatment laser beam (B) in the eye tissue (91); and
generating (S26), one or more feedback signals depending on the eye tissue modification score.

17. A computer program product comprising computer program code which, when the computer program code is executed by an electronic circuit (5) of an ophthalmological laser treatment device (1), causes the electronic circuit (5) to perform the steps of the method according to claim 16.
